# EUROPEAN PATENT SPECIFICATION

(11) **EP 3 528 801 B1**
(45) Date of publication and mention of the grant of the patent: **17.07.2024**
(21) Application number: 17862326.0
(22) Date of filing: 20.10.2017
(51) Int. Cl.: A61K 31/19, A61P 3/04, A23L 33/10, A23L 33/135, A61K 35/742, A61P 21/00, A61P 43/00

(54) **COMPOSITIONS AND METHODS OF USE OF BETA-HYDROXY-BETA-METHYLBUTYRATE (HMB) AND PROBIOTICS**
ZUSAMMENSETZUNGEN UND VERFAHREN ZUR VERWENDUNG VON BETA-HYDROXY-BETA-METHYLBUTYRAT (HMB) UND PROBIOTIKA
COMPOSITIONS ET MÉTHODES D'UTILISATION DE BETA-HYDROXY-BETA-MÉTHYLBUTYRATE (HMB) ET DE PROBIOTIQUES

(30) Priority: 21.10.2016 US 201662411200 P
(43) Date of publication of application: 28.08.2019
(73) Proprietor: Metabolic Technologies, LLC, Ames, IA 50010 (US)
(72) Inventor: HOFFMAN, Jay, Oviedo FL 32766 (US); RATHMACHER, John, Story City IA 50248 (US)
(74) Representative: PATERIS Patentanwälte PartmbB
(86) International application number: PCT/US2017/057549
(87) International publication number: WO 2018/075867

(56) References cited:
- WO-A1-2014/099904
- WO-A1-2014/144458
- WO-A2-2015/105981
- WO-A2-2015/105981
- US-A1- 2010 179 112
- US-A1- 2016 021 921
- US-A1- 2016 037 815
- US-A1- 2016 037 815
- US-A1- 2016 038 457
- US-A1- 2016 066 610
- US-A1- 2016 066 610
- RALF JÄGER ET AL: "Probiotic Bacillus coagulans GBI-30, 6086 reduces exercise-induced muscle damage and increases recovery", PEERJ, vol. 4, 21 July 2016 (2016-07-21), pages 1 - 14, XP055478229, DOI: 10.7717/peerj.2276
- ANONYMOUS: "Study NCT02762968 Submitted Date: May 3, 2016 (v1)", 3 May 2016 (2016-05-03), XP055699581, Retrieved from the Internet <URL:https://clinicaltrials.gov/ct2/history/NCT02762968?V_1=View#StudyPageTop> [retrieved on 20200529]
- YFTACH GEPNER ET AL: "Combined effect of Bacillus coagulans GBI-30, 6086 and HMB supplementation on muscle integrity and cytokine response during intense military training", JOURNAL OF APPLIED PHYSIOLOGY, vol. 123, no. 1, 1 July 2017 (2017-07-01), US, pages 11 - 18, XP055699575, ISSN: 8750-7587, DOI: 10.1152/japplphysiol.01116.2016
- HOFFMAN ET AL.: "B-Hydroxy-B-methylbutyrate attenuates cytokine response during sustained military training", NUTRITION RESEARCH, vol. 36, 21 February 2016 (2016-02-21), pages 553 - 563, XP055478225
- JAGER ET AL.: "Probiotic Bacillus coagulans GBI-30, 6086 reduces exercise-induced muscle damage and increases recovery", PEERJ, vol. 4, 21 July 2016 (2016-07-21), pages 1 - 14, XP055478229
- SHREERAM ET AL.: "The Relative Bioavailability of the Calcium Salt of B-Hydroxy-B-Methylbutyrate Is Greater Than That of the Free Fatty Acid Form in Rats", THE JOURNAL OF NUTRITION, vol. 144, 1 October 2014 (2014-10-01), pages 1549 - 1555, XP055308607

## Description

### Background of the Invention

### 1. Field

The present disclosure relates to a composition comprising β-hydroxy-β-methylbutyrate (HMB) and probiotics and non-therapeutic methods of using the composition to attenuate inflammatory cytokine markers and/or maintain muscle integrity.

### 2. Background

### HMB

Alpha-ketoisocaproate (KIC) is the first major and active metabolite of leucine. A minor product of KIC metabolism is β-hydroxy-β-methylbutyrate (HMB). HMB has been found to be useful within the context of a variety of applications. Specifically, in U.S. Patent No. 5,360,613 (Nissen), HMB is described as useful for reducing blood levels of total cholesterol and low-density lipoprotein cholesterol. In U.S. Patent No. 5,348,979 (Nissen et al.), HMB is described as useful for promoting nitrogen retention in humans. U.S. Patent No. 5,028,440 (Nissen) discusses the usefulness of HMB to increase lean tissue development in animals. Also, in U.S. Patent No. 4,992,470 (Nissen), HMB is described as effective in enhancing the immune response of mammals. U.S. Patent No. 6,031,000 (Nissen et al.) describes use of HMB and at least one amino acid to treat disease-associated wasting.

The use of HMB to suppress proteolysis originates from the observations that leucine has protein-sparing characteristics. The essential amino acid leucine can either be used for protein synthesis or transaminated to the α-ketoacid (α-ketoisocaproate, KIC). In one pathway, KIC can be oxidized to HMB and this accounts for approximately 5% of leucine oxidation. HMB is superior to leucine in enhancing muscle mass and strength. The optimal effects of HMB can be achieved at 3.0 grams per day when given as calcium salt of HMB, or 0.038g/kg of body weight per day, while those of leucine require over 30.0 grams per day.

Once produced or ingested, HMB appears to have two fates. The first fate is simple excretion in urine. After HMB is fed, urine concentrations increase, resulting in an approximate 20-50% loss of HMB to urine. Another fate relates to the activation of HMB to HMB-CoA. Once converted to HMB-CoA, further metabolism may occur, either dehydration of HMB-CoA to MC-CoA, or a direct conversion of HMB-CoA to HMG-CoA, which provides substrates for intracellular cholesterol synthesis. Several studies have shown that HMB is incorporated into the cholesterol synthetic pathway and could be a source for new cell membranes that are used for the regeneration of damaged cell membranes. Human studies have shown that muscle damage following intense exercise, measured by elevated plasma CPK (creatine phosphokinase), is reduced with HMB supplementation within the first 48 hrs. The protective effect of HMB lasts up to three weeks with continued daily use. Numerous studies have shown an effective dose of HMB to be 3.0 grams per day as CaHMB (calcium HMB) (~38 mg •kg body weight⁻¹•day⁻¹). HMB has been tested for safety, showing no side effects in healthy young or old adults. HMB in combination with L-arginine and L-glutamine has also been shown to be safe when supplemented to AIDS and cancer patients.

Recently, HMB free acid, a new delivery form of HMB, has been developed. This new delivery form has been shown to be absorbed quicker and have greater tissue clearance than CaHMB. The new delivery form is described in U.S. Patent Publication Serial No. 20120053240.

HMB has been demonstrated to enhance recovery and attenuate muscle damage from high intensity exercise. HMB attenuates the depression of protein synthesis with TNF-alpha and decreases protein degradation associated with TNF.

In studies examining intense physical activity with minimal recovery, such as that encountered by soldiers during sustained combat operations, the use of HMB supplementation may mitigate the deleterious effects associated with this physical stress. Significant decrements in body mass, strength and power have been reported in soldiers during sustained military operations. These stresses are also associated with significant elevations in inflammatory cytokine marker. A recent field study demonstrated that when HMB is supplemented by soldiers for three weeks during intense training, including simulated combat, the inflammatory response was attenuated, and accompanied by a maintenance of muscle integrity as determined through diffusion tensor imaging. These results were consistent with other investigations reporting that short (e.g., 4 days) and long (e.g., 12-weeks) duration HMB supplementation can attenuate the cytokine response to a muscle damaging protocol.

### Probiotics

The use of probiotics as a dietary supplement has become very popular in the past few years for the prevention and treatment of a variety of diseases. Probiotics are live bacteria that are suggested to be beneficial for improving digestive health and immune function, while decreasing inflammation. It is thought that probiotics, such as *Bacillus coagulans,* can enhance enzymatic digestion of foods within the gut resulting in greater absorption of nutrients.

It has been surprisingly and unexpectedly discovered that HMB in combination with probiotics attenuates inflammatory cytokine markers and maintains muscle integrity. A reduction in the cytokine response to intense training has often been used to indicate a more favorable recovery from high intensity training. The combination of HMB and probiotics is synergistic such that the combination results in enhanced absorption of HMB and a greater increase in circulating HMB as compared to administration of HMB alone. This synergism between HMB and probiotics is demonstrated by improved attenuation of inflammatory cytokine markers and muscle integrity as compared to the effects of HMB when administered alone.

WO 2014/144458 A1 describes a nutritional composition comprising from about 1 g to about 3 g of HMB and at least one probiotic. It further describes such a composition for use in increasing muscle protein synthesis, or decreasing muscle protein degradation, or reducing muscle necrosis or apoptosis.

US 2016/037815 A1 describes a nutritional composition comprising from about 0.2 g to about 4 g of HMB and at least one probiotic. It further describes such a composition for use in maintaining muscle integrity.

US 2016/066610 A1 describes a nutritional composition comprising from about 0.5 g to about 3 g of HMB and at least one probiotic. It further describes such a composition for use in maintaining muscle function.

WO 2015/105981 A2 describes a nutritional composition comprising from about 0.5 g to about 3 g of HMB and at least one probiotic.

WO 2014/099904 A1 describes a nutritional composition comprising from about 0.4 g to about 4 g of HMB and at least one probiotic.

Jäger et al., PeerJ, vol. 4, 2016, pages 1-14 describes the utility of the probiotic *Bacillus coagulans* in reducing exercise-induced muscle damage.

The study protocol of clinical study NCT02762968, submitted May 3, 2016 (v1), describes a combination of capsules providing 3 g calcium HMB per day plus *Bacillus coagulans* GBI-30, 6086 (BC30) mixed in water. It further describes that muscle damage markers will be assessed, but does not disclose any study results.

### Summary of the Invention

The invention is set out in the appended set of claims.

### Summary of the Disclosure

The composition, the medical uses of the composition and the methods described in the following summary of the disclosure are not covered by the claimed invention.

The disclosure provides a composition for use in maintaining muscle integrity.

The disclosure provides a composition for use in attenuating inflammatory cytokine markers.

The disclosure provides methods of administering a composition for use in maintaining muscle integrity.

The disclosure provides methods of administering a composition for use in attenuating inflammatory cytokine markers.

The disclosure provides methods of improving the absorption of HMB by adding probiotics to a composition containing HMB.

The disclosure provides methods of increasing the time that HMB is in the bloodstream by adding probiotics to a composition containing HMB.

These and other aspects of the present disclosure will become apparent to those skilled in the art upon reference to the following specification, drawings, and claims.

The present disclosure intends to overcome the difficulties encountered heretofore. To that end, the disclosure provides a composition comprising HMB and probiotics. The composition is administered to a subject in need thereof. All methods comprise administering to the animal HMB and probiotics. The subjects included include humans and non-human mammals. The composition is consumed by a subject in need thereof.

### Brief Description of the Drawings

Figure 1 depicts changes in the inflammatory cytokine response to intense military training following forty (40) days of supplementation.
Figure 2 depicts changes in DTI measure following forty (40) days of supplementation.

### Detailed Description

The present invention relates to a non-therapeutic use of a composition comprising from about 0.5 g to about 30 g of β-hydroxy-β-methylbutyric acid (HMB) and at least one probiotic for use in maintaining muscle integrity in an animal in need thereof, wherein the composition is to be administered to the animal, wherein the animal is a human or a non-human mammal, wherein the probiotic is *Bacillus coagulans.*

It has been surprising and unexpectedly discovered that probiotics and HMB have a synergistic relationship. Use of compositions containing HMB and probiotics results in attenuation of inflammatory cytokine markers and maintenance of muscle integrity, and these effects are seen in greater amounts than administration of HMB alone. The combination of HMB and probiotics is synergistic such that the combination results in enhanced absorption of HMB and a greater increase in circulating HMB as compared to administration of HMB alone.

### HMB

β-hydroxy-β-methylbutyric acid, or β-hydroxy-isovaleric acid, can be represented in its free acid form as (CH₃)₂(OH)CCH₂COOH. The term "HMB" refers to the compound having the foregoing chemical formula, in both its free acid and salt forms. While any form of HMB can be used within the context of the present invention, preferably HMB is selected from the group comprising a free acid, a salt, an ester, and a lactone. HMB esters include methyl and ethyl esters. HMB lactones include isovalaryl lactone. HMB salts include sodium salt, potassium salt, chromium salt, calcium salt, magnesium salt, alkali metal salts, and earth metal salts.

Methods for producing HMB are well-known in the art. For example, HMB can be synthesized by oxidation of diacetone alcohol. One suitable procedure is described by Coffman et al., J. Am. Chem. Soc. 80: 2882-2887 (1958). As described therein, HMB is synthesized by an alkaline sodium hypochlorite oxidation of diacetone alcohol. The product is recovered in free acid form, which can be converted to a salt. For example, HMB can be prepared as its calcium salt by a procedure similar to that of Coffman et al. (1958) in which the free acid of HMB is neutralized with calcium hydroxide and recovered by crystallization from an aqueous ethanol solution. The calcium salt of HMB is commercially available from Metabolic Technologies, Ames, Iowa.

### Calcium β-hydroxy-β-methytbutyrate (HMB) Supplementation

More than 2 decades ago, the calcium salt of HMB was developed as a nutritional supplement for humans. Studies have shown that 38 mg of CaHMB per kg of body weight appears to be an efficacious dosage for an average person.

The molecular mechanisms by which HMB decreases protein breakdown and increases protein synthesis have been reported. Eley et al conducted *in vitro* studies which have shown that HMB stimulates protein synthesis through mTOR phosphorylation. Other studies have shown HMB decreases proteolysis through attenuation of the induction of the ubiquitin-proteosome proteolytic pathway when muscle protein catabolism is stimulated by proteolysis inducing factor (PIF), lipopolysaccharide (LPS), and angiotensin II. Still other studies have demonstrated that HMB also attenuates the activation of caspases-3 and -8 proteases.

### HMB Free Acid form

In most instances, the HMB utilized in clinical studies and marketed as an ergogenic aid has been in the calcium salt form. Recent advances have allowed the HMB to be manufactured in a free acid form for use as a nutritional supplement. Recently, a new free acid form of HMB was developed, which was shown to be more rapidly absorbed than CaHMB, resulting in quicker and higher peak serum HMB levels and improved serum clearance to the tissues.

HMB free acid may therefore be a more efficacious method of administering HMB than the calcium salt form, particularly when administered directly preceding intense exercise. One of ordinary skill in the art, however, will recognize that this current invention encompasses HMB in any form.

HMB in any form may be incorporated into the delivery and/or administration form in a fashion so as to result in a typical dosage range of about 0.5 grams HMB to about 30 grams HMB.

Any suitable dose of HMB can be used within the context of the present disclosure. Methods of calculating proper doses are well known in the art. The dosage amount of HMB can be expressed in terms of corresponding mole amount of Ca-HMB. The dosage range within which HMB may be administered orally or intravenously is within the range from 0.01 to 0.2 grams HMB (Ca-HMB) per kilogram of body weight per 24 hours. For adults, assuming body weights of from about 100 to 200lbs., the dosage amount orally or intravenously of HMB (Ca-HMB basis) can range from 0.5 to 30 grams per subject per 24 hours.

### Probiotics

Probiotics, such as *Bacillus coagulans* (BC30), have many health benefits including improving digestive health and immune function and decreasing inflammation. In addition, previous studies have suggested that BC30 can enhance protein absorption. Any probiotic is suitable for use in the composition described herein. Appropriate amounts of *Bacillus coagulans* will be understood by those of skill in the art. A typical composition of the present disclosure will contain in a one gram dosage formulation from 2×10⁵ to 10¹⁰ colony forming units of viable bacteria or bacterial spore (in the case of *Bacillus coagulans*)*.* In other embodiments, the amount of bacteria include probiotics at a concentration of from about 1 × 10⁴ to about 1 × 10¹² viable bacteria. The *Bacillus coagulans* bacteria are in the form of spores, vegetative cells, or a combination thereof. Although *Bacillus coagulans* was used in the experimental examples, the disclosure is not limited to this particular *Bacillus* species. Any species of probiotic bacteria can be used in the compositions and methods of the present disclosure.

When the composition is administered orally in an edible form, the composition is preferably in the form of a dietary supplement, foodstuff or pharmaceutical medium, more preferably in the form of a dietary supplement or foodstuff. Any suitable dietary supplement or foodstuff comprising the composition can be utilized within the context of the present invention. One of ordinary skill in the art will understand that the composition, regardless of the form (such as a dietary supplement, foodstuff or a pharmaceutical medium), may include amino acids, proteins, peptides, carbohydrates, fats, sugars, minerals and/or trace elements.

In order to prepare the composition as a dietary supplement or foodstuff, the composition will normally be combined or mixed in such a way that the composition is substantially uniformly distributed in the dietary supplement or foodstuff. Alternatively, the composition can be dissolved in a liquid, such as water.

The composition of the dietary supplement may be a powder, a gel, a liquid or may be tabulated or encapsulated.

Although any suitable pharmaceutical medium comprising the composition can be utilized within the context of the present invention, preferably, the composition is combined with a suitable pharmaceutical carrier, such as dextrose or sucrose.

Furthermore, the composition of the pharmaceutical medium can be intravenously administered in any suitable manner. For administration via intravenous infusion, the composition is preferably in a water-soluble non-toxic form. Intravenous administration is particularly suitable for hospitalized patients that are undergoing intravenous (IV) therapy. For example, the composition can be dissolved in an IV solution (e.g., a saline or glucose solution) being administered to the patient. Also, the composition can be added to nutritional IV solutions, which may include amino acids, glucose, peptides, proteins and/or lipids. The amounts of the composition to be administered intravenously can be similar to levels used in oral administration. Intravenous infusion may be more controlled and accurate than oral administration.

Methods of calculating the frequency by which the composition is administered are well-known in the art and any suitable frequency of administration can be used within the context of the present invention (e.g., one 6 g dose per day or two 3 g doses per day) and over any suitable time period (e.g., a single dose can be administered over a five minute time period or over a one hour time period, or, alternatively, multiple doses can be administered over an extended time period). The composition can be administered over an extended period of time, such as weeks, months or years.

Any suitable dose of HMB and probiotics can be used within the context of the present disclosure. Methods of calculating proper doses are well known in the art.

It will be understood by one of ordinary skill in the art that HMB and probiotics do not have to be administered in the same composition to perform the disclosed methods. Stated another way, separate capsules, pills, mixtures, etc. of probiotics and of HMB may be administered to a subject to carry out the disclosed methods.

The term administering or administration includes providing a composition to a mammal, consuming the composition and combinations thereof.

### Experimental Examples

The following examples will illustrate the invention in further detail. It will be readily understood that the composition of the present disclosure, as generally described and illustrated in the Examples herein, could be synthesized in a variety of formulations and dosage forms.

### METHODS

### Participants

Twenty-six male soldiers from an elite combat unit of the Israel Defense Forces (IDF) volunteered to participate in this double-blind, parallel design study. Following an explanation of all procedures, risks and benefits, each participant provided his informed consent to participate in the study. The Helsinki committee of the IDF Medical Corp and by the Medical Ethics Board and the Helsinki Committee of Soroka Medical Center approved this research study. Participants were not permitted to use any additional dietary supplementation, and did not consume any androgens or other performance enhancing drugs. Screening for performance enhancing drug use and additional supplementation was accomplished via a health questionnaire completed during participant recruitment. Soldiers were from the same unit and were randomly assigned to one of two groups: CaHMB with BC30 (CaHMBBC30; n = 9; 20.5 ± 0.8 y; 1.75 ± 0.09 m; 75.4 ± 9.6 kg) or CaHMB with placebo (CaHMBPL, n = 9; 19.1 ± 3.4 y;1.73 ± 0.05 m; 71.4±6.4 kg). A third group of participants from the same unit, who were interested in participating in the study, but were not interested in consuming a supplement agreed to serve as a control group (CTL; n = 8; 20.4 ± 0.7 y; 1.73 ± 0.05 m; 68.6±5.3 kg).

### Study protocol

During the 40-day intervention period, all participants performed the same daily protocol. During the first 28 days soldiers were garrisoned on base and participated in the same advanced military training tasks that included combat skill development and conditioning including 90 minutes of intense hand-to-hand combat (krav-maga) training five times a week. The physical training included on average two 5-km runs per week. During weeks 5 and 6 soldiers were in the field and navigated between 25 km - 30 km per night in difficult terrain carrying approximately 35 kg of equipment on their back (equating to approximately 40% of participant's body mass). The duration of the navigational exercise lasted between 5 - 8 hours per evening. During the last evening of training (day 40), the soldiers also performed an additional 5 km stretcher carry following the navigational training. During the final two weeks of training soldiers slept between 5 - 8 hours per night. All assessments (blood draws and magnetic resonance imaging [MRI]) were conducted in a single day prior to (PRE) and approximately 12-hours following the final supplement consumption (on day 40) (POST). All assessments were performed in the same order at both PRE and POST.

### Supplementation Protocol

Participants in both CaHMBBC30 and CaHMBPL ingested 1.0 g of CaHMB three times per day for a total daily consumption of 3 g. Each serving consisted of 4 capsules (250 mg of CaHMB) consumed during morning, noon and evening meals. CaHMB was obtained from Metabolic Technologies Inc. (Ames, IA, USA). The probiotic supplement (*Bacillus coagulans* GBI-30, 6086) was provided by Ganeden Biotech, Inc (Mayfield Hts, OH, USA). Each serving contained 2.0 × 10¹⁰ colony forming units. Participants consumed one serving per day (morning meal). The placebo was provided by the manufacturer and matched in appearance, weight and taste to the active product. Both the placebo and active product were provided in powder form and mixed in water (~250 ml) prior to ingestion. Participants in CaHMBBC30 and CaHMBPL were provided with two 20-day supplies of HMB and PL. Participants were required to return all used and unused packets at the end of each 20 day period.

### Blood measurements

Resting blood samples were obtained prior to each testing session. All blood samples were obtained following a 15-min equilibration period. These blood samples were obtained from an antecubital arm vein using a 20-gauge disposable needle equipped with a Vacutainer^{®} tube holder (Becton Dickinson, Franklin Lakes, NJ). Each participant's blood samples were obtained at the same time of day during each session following an overnight fast. All blood samples were collected into two Vacutainer^{®} tubes, one containing no anti-clotting agent and the second containing K₂EDTA. The blood in the first tube was allowed to clot at room temperature for 30-min and subsequently centrifuged at 3,000×g for 15-min along with the remaining whole blood from the second tube. The resulting plasma and serum was placed into separate 1.8-ml microcentrifuge tubes and frozen at -80°C for later analysis.

### Biochemical analysis

Serum concentrations of creatine kinase (CK) and lactate dehydrogenase (LDH) were analyzed using a commercially available kinetic assay (Sekisui Diagnostics, Charlottetown, PE, Canada; Sigma-Aldrich, St. Louis, MO, USA), per manufacturer's instructions. Plasma concentrations of cytokines and chemokines included granulocyte-macrophage colony stimulating factor (GM-CSF), fractalkine (CX3CL1), interferon-gamma (INF-γ), interluekin-1beta (IL-1β), interleukin-2 (IL-2), interleukin-6 (IL-6), interleukin-8 (IL-8), interleukin-10 (IL-10), and tumor necrosis factor-alpha (TNF-α) were analyzed via multiplex assay, using the human cytokine/chemokine panel one (EMD Millipore, Billerica, MA, USA). In addition, plasma HMB concentrations were analyzed by gas chromatography-mass spectrometry and performed by Metabolic Technologies Inc. using methods previously described. All samples were thawed once and analyzed in duplicate by the same technician using a BioTek Eon spectrophotometer for CK and LDH concentrations (BioTek, Winooski, VT, USA), and MagPix for cytokine and chemokine concentrations (EMD Millipore). Mean intra-assay variability for all assays was below 10%.

### Magnetic resonance imaging (MRI)

Changes in muscle integrity of the rectus femoris (RF) and vastus lateralis (VL) were assessed using MRI. Due to logistical considerations (element of time), it was determined *a priori* that the primary focus of the study was to compare the effect of BC30 co-administered with CaHMB to CaHMB only, as such only soldiers in CaHMBBC30 and CaHMBPL were assessed with MRI. Muscle integrity was determined through densor tensor imaging (DTI). DTI is a sensitive MRI technique to assess subclinical signs of muscle injury. DTI assessment is predicated on cell membranes and other structures constraining water diffusion. Water movement can be evaluated by determining the three orthogonal directions of water diffusion, called eigenvectors, and their intensities, called eigenvalues. From the three eigenvalues (λ1, λ2 and λ3), parameters such as fractional anisotropy (FA) and apparent diffusion coefficients (ADC) can be calculated to evaluate the character of water diffusion in a voxel. These measures have been demonstrated to provide information about the integrity of skeletal muscle.

The MRI data were obtained using a 3.0 Tesla whole-body imager (Ingenia, Philips Medical Systems, Best, The Netherlands). During each measure participants were placed supine in the scanner and imaged using phased-array surface coils. A position 20cm above the patella was chosen as the image center and marked using an oil capsule. All scans were planned axially and consisted of 40 slices of 4mm width for a foot-head coverage of 160mm, and a field-of-view of 290 × 280 mm (RL×AP). Three image acquisitions were performed. A T1w DIXON was used for anatomical reference, a T2w Turbo spin-echo to assess any structural damage to the muscle, and a diffusion tensor imaging (DTI) sequence for muscle fiber tracking. The sequence parameters that were used have been previously published.

Fat suppression (SPAIR - spectrally selective adiabatic inversion recovery) was used for the T2-TSE and DTI scans. The DTI sequence was a 2D-EPI sequence imaged in two packages. The b-value was 400 sec/mm2 and imaged in 15 unique directions. Muscle fiber tracking analysis was calculated by using the Philips 'FiberTrak' software. An ROI (region of interest) was hand drawn for RF and VL on slices 15 and 25. The software then was allowed to delineate the muscle fibers using an algorithm that eliminated tracks if the FA was less than 0.1, if the change in angle was greater than 27° or if the fiber length was less than 10mm. The same investigator performed all assessments.

### Statistical analyses

Analyses of covariance (ANCOVA) was used to analyze all MRI and blood dependent variables (muscle damage markers and cytokines). PRE and POST values were used as the covariate and dependent variable, respectively. In the event of a significant f ratio, LSD post hoc pairwise comparisons were used to examine the differences among the groups. Results of the ANCOVA were also converted to change from PRE. An alpha level of p ≤ 0.05 was considered statistically significant for all comparisons. All data are reported as mean ± SD unless otherwise noted. Statistical analysis was performed with SPSS (IBM Statistics for Windows, Version 23.0; Armonk, NY: IBM Corp).

### RESULTS

Of the 26 solders that participated in this trial, 25 completed the intervention. The only participant who withdrew from the study was injured during training. No side effects associated with supplementation were reported during the study. Based on CaHMB and BC30 consumption (determined by the number of capsules and BC-30 packets returned) compliance for supplementation was 95.0 ± 3.0% among the two CaHMB groups. An additional measure for study compliance was conducted by analyzing plasma HMB concentrations at PRE and POST for participants in both supplement groups. Significant elevations (p = 0.010) were noted in plasma HMB concentrations from PRE (3.28 ± 0.73 nmol·L⁻¹) to POST (34.1 ± 43.9 nmol·L⁻¹) assessments.

### Blood data

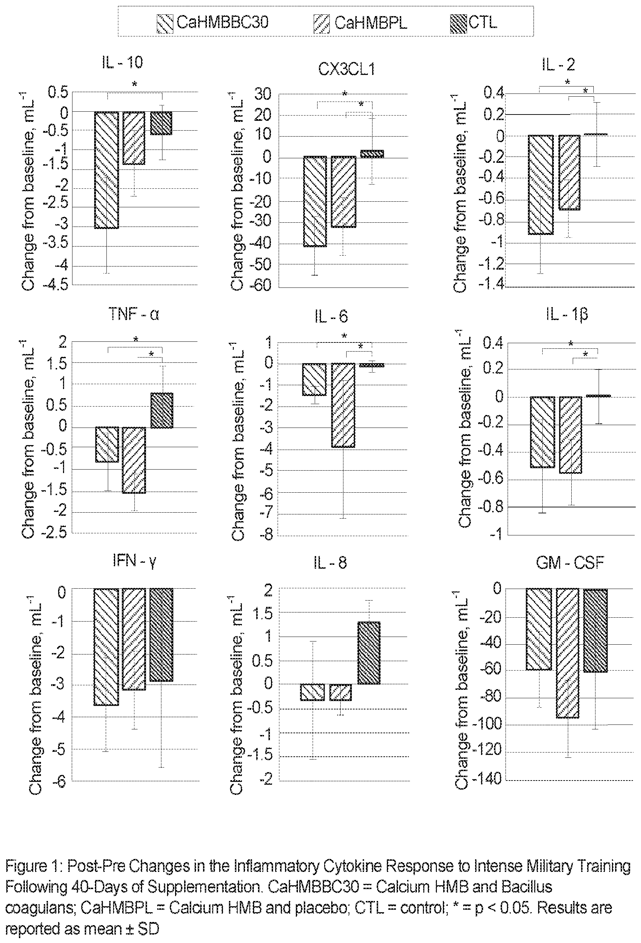

Circulating concentrations of inflammatory cytokines can be observed in Table 1. In addition, comparisons of the change from PRE between groups are depicted in Figure 1. A significant interaction (F = 6.48, p = 0.006) was observed for changes in circulating TNF-α concentrations. Plasma TNF-α concentrations at POST was significantly lower for CaHMBBC30 and CaHMBPL than CTL (p = 0.019 and p = 0.002, respectively). However, no differences were noted between CaHMBBC30 and CaHMBPL (p = 0.290). A significant interaction (F = 4.70, p = 0.025) was also noted between the groups for changes in plasma CX3CL1 concentrations. Changes in CX3CL1 concentrations were significantly lower for CaHMBBC30 and CaHMBPL than CTL (p = 0.044 and p = 0.011, respectively). No differences in CX3CL1 concentrations (P = 0.687) were noted between CaHMBBC30 and CaHMBPL. A significant interaction (F = 6.93, p = 0.006) was noted for changes in plasma IL-1β concentrations. IL-1β concentrations were significantly attenuated for both CaHMBBC30 and CaHMBPL compared to CTL (p = 0.005 and p = 0.004, respectively). No differences (p = 0.878) were observed between CaHMBBC30 and CaHMBPL. A significant interaction (F = 4.96, p = 0.019) was also found for plasma IL-2 concentrations. Circulating IL-2 concentrations for CaHMBBC30 and CaHMBPL were significantly attenuated at POST compared to CTL (p = 0.007 and p = 0.029, respectively). No difference (p = 0.584) was noted between CaHMBBC30 and CaHMBPL. A significant interaction (F=7.99, p = 0.005) was also observed in changes in plasma IL-6 concentrations. Plasma IL-6 concentrations were significantly attenuated in CaHMBBC30 and CaHMBPL compared to CTL (p = 0.002 and p = 0.018, respectively). No difference (p = 0.467) in the IL-6 response at POST was noted between CaHMBBC30 and CaHMBPL. A significant interaction (F=3.72, p = 0.041) was also observed in changes in plasma IL-10 concentrations. A significant difference (p = 0.013) was seen between CaHMBBC30 and CTL. No other significant differences were noted. No significant interactions were observed for changes in INF-γ (F=1.25, p = 0.31), IL-8 (F = 1.49, p = 0.25) or GM-CSF (F= 0.71, p = 0.50) concentrations.

**Table 1. The effect of 40 day of intense military training on cytokine concentrations across study groups.**

| | | CaHMBBC30 n=9 | CaHMBPL n=9 | CTL n=8 | p value |
|---|---|---|---|---|---|
| TNF-α | Pre | 2.48±1.95 | 2.33±1.31 | 3.08±2.30 | 0.006 |
| | Post | 1.67±2.01 | 0.81±0.36 | 3.86±2.21 | |
| CX3CL1 | Pre | 106±63.2 | 81.6±52.3 | 115±64.1 | 0.025 |
| | Post | 65.1±42.9 | 70.7±29.9 | 118±63.1 | |
| IL-1β | Pre | 0.95±1.09 | 0.89±0.85 | 1.20±1.03 | 0.006 |
| | Post | 0.40±0.41 | 0.42±0.37 | 1.21±0.81 | |
| IL-2 | Pre | 1.58±0.93 | 1.35±1.34 | 2.21±1.81 | 0.019 |
| | Post | 0.66±0.34 | 0.80±0.87 | 2.21±1.82 | |
| IL-6 | Pre | 1.87±0.89 | 7.63±17.85 | 2.48±1.42 | 0.005 |
| | Post | 0.51±0.24 | 5.20±10.68 | 2.34±1.60 | |
| IL-10 | Pre | 6.85±4.85 | 4.96±4.01 | 9.92±9.77 | 0.041 |
| | Post | 3.89±2.82 | 3.62±2.64 | 9.37±8.79 | |
| IFN-γ | Pre | 9.45±6.73 | 11.3±8.31 | 18.2±13.5 | 0.31 |
| | Post | 5.81±4.75 | 8.11±6.75 | 15.4±11.2 | |
| IL-8 | Pre | 4.14±6.28 | 2.58±1.05 | 2.53±1.82 | 0.25 |
| | Post | 3.84±4.11 | 2.27±0.92 | 3.83±2.86 | |
| GM-CSF | Pre | 232±323 | 170±148.9 | 217±236 | 0.50 |
| | Post | 173±338 | 75.6±81.6 | 156±144 | |

| | | | | | |
|---|---|---|---|---|---|
| CaHMBBC30 = Calcium HMB and Bacillus coagulans; CaHMBPL = Calcium HMB and placebo; CTL = control. All data are reported as mean ± SD. ANCOVA test was used to assess differences between groups. | | | | | |

Analysis of muscle damage markers revealed no significant interactions between the groups for plasma LDH (F = 0.15, p = 0.86) or CK concentrations (F = 0.17, p = 0.84). No changes in LDH concentrations were noted from PRE (537.7 ± 86.1 IU·L⁻¹) to POST (567.5 ± 87.4 IU·L⁻¹) in the groups combined. In addition, no change was noted in CK concentrations from PRE (225.4 ± 79.8 IU·L⁻¹) to POST (377.6 ± 230.2 IU L-1) in the groups combined.

### DTI

Comparisons of FA and ADC assessments between CaHMBBC30 and CaHMBPL can be observed in Table 2.

**Table 2: MRI and DTI measures in β-Hydroxy-β-methylbutyrate with and without probiotics (BC30) in response to intense military training**

| | | Pre | Post | Effect over time P value |
|---|---|---|---|---|
| **Rector femoris** | | | | |
| Fractional Anisotropy | CaHMBBC30 | 0.24±0.05 | 0.20±0.02 | 0.014 |
| | CaHMBPL | 0.23±0.02 | 0.19±0.02 | |
| Apparent Diffusion Coefficient (× 10-3 mm²/s) | CaHMBBC30 | 1.79±0.09 | 1.72±0.09 | 0.69 |
| | CaHMBPL | 1.68±0.06 | 1.77±0.04 | |

| **Vastus lateralis** | | | | |
|---|---|---|---|---|
| Fractional Anisotropy | CaHMBBC30 | 0.20±0.03 | 0.21±0.02 | 0.23 |
| | CaHMBPL | 0.20±0.01 | 0.19±0.01 | |
| Apparent Diffusion Coefficient (× 10-3 mm²/s) | CaHMBBC30 | 1.73±0.05 | 1.71±0.05 | 0.86 |
| | CaHMBPL | 1.73±0.06 | 1.75±0.12 | |

| | | | | |
|---|---|---|---|---|
| All data in the table are reported as mean±SD. Paired T-test was used to assess changes over time for the both groups. CaHMBBC30 = Calcium HMB and Bacillus coagulans; CaHMBPL = Calcium HMB and placebo; CTL = control. | | | | |

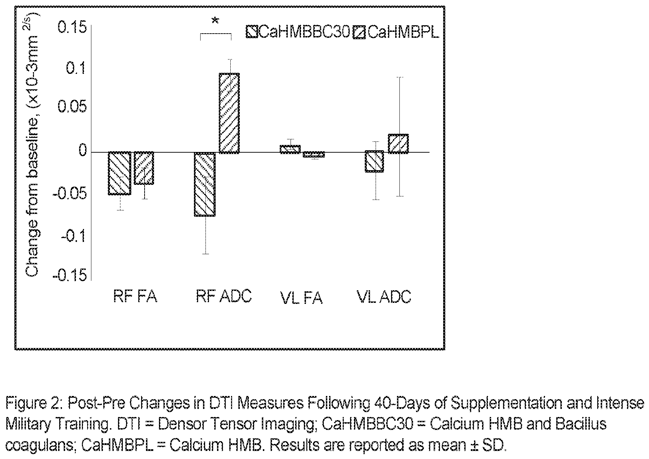

In addition, comparisons of the change from PRE between groups is depicted in Figure 2. No significant difference (F = 0.315, p= 0.587) in FA was observed between CaHMBBC30 and CaHMBPL in the RF, however, when collapsed across groups a significant decrease was noted from PRE to POST. A significant difference (F = 7.198, p = 0.023) in ADC was noted between the groups in RF. Participants in CaHMBBC30 experienced a decrease in ADC, while participants in CaHMBPL experienced an increase. No significant differences between the groups were noted in the VL for either FA (F = 2.95, p = 0.117) or ADC (F = 1.886, p = 0.200).

The results of this study indicate that 40 days of HMB supplementation with and without *Bacillus coagulans* can attenuate inflammatory cytokine markers during highly intense military training. This combination appeared to attenuate the IL-10 response compared to the control. In addition, the combination of CaHMB and BC30 provided a significant benefit compared to CaHMB alone in maintaining muscle integrity, as indicated by a decrease in apparent diffusion coefficient (ADC) for the rectus femoris (RF).

Plasma HMB for CaHMBPC30, CaHMBPL and CTL were 50.6 ± 15.6 nmol·L⁻¹, 15.6 ± 28.0 nmol·L⁻¹ and 3.3 ± 0.9 nmol·L⁻¹, respectively. The greater HMB concentrations observed for CaHMBBC30 indicates that BC30 has enhanced absorption capability.

Measures of muscle integrity were conducted using DTI, which is considered to be a sensitive method of assessing subclinical signs of muscle injury. DTI measures the diffusion of water molecules and the direction of their movement in a three-dimensional muscle microstructure. In healthy tissue the integrity of the structure results in a barrier to diffusion. Fractional anisotropy (FA) represents the increase in diffusivity into tissue following trauma, while apparent diffusion coefficient (ADC) reflects the degree of diffusion in each direction of the muscle by the length of its axis. A decrease in FA, and an increase in ADC, represent a disruption to the integrity of the muscle indicating greater diffusion. Previously, we reported significant decreases in FA in both the RF and semitendinosus for placebo group only, and a likely increase in ADC of the VL in the supplement group indicating that HMB provided in its free acid form may enhance muscle integrity during intense military training. However, the data show that the addition of BC30 to CaHMB provided a synergistic effect for maintaining muscle integrity resulting in a greater degree of muscle protection than that offered by CaHMB alone.

The foregoing description and drawings comprise illustrative embodiments of the present invention. The foregoing embodiments and the methods described herein may vary based on the ability, experience, and preference of those skilled in the art. Merely listing the steps of the method in a certain order does not constitute any limitation on the order of the steps of the method.

### References

1. Gonzalez AM, Fragala MS, Jajtner AR, et al. Effects of beta-hydroxy-beta-methylbutyrate free acid and cold water immersion on expression of CR3 and MIP-1beta following resistance exercise. American journal of physiology Regulatory, integrative and comparative physiology. 2014; 306: R483-9.
2. Gonzalez AM, Stout JR, Jajtner AR, et al. Effects of beta-hydroxy-beta-methylbutyrate free acid and cold water immersion on post-exercise markers of muscle damage. Amino acids. 2014; 46: 1501-11.
3. Kraemer WJ, Hatfield DL, Volek JS, et al. Effects of amino acids supplement on physiological adaptations to resistance training. Medicine and science in sports and exercise. 2009; 41: 1111-21.
4. Townsend JR, Fragala MS, Jajtner AR, et al. beta-Hydroxy-beta-methylbutyrate (HMB)-free acid attenuates circulating TNF-alpha and TNFR1 expression postresistance exercise. Journal of applied physiology. 2013; 115: 1173-82.
5. van Someren KA, Edwards AJ, Howatson G. Supplementation with beta-hydroxy-beta-methylbutyrate (HMB) and alpha-ketoisocaproic acid (KIC) reduces signs and symptoms of exercise-induced muscle damage in man. International journal of sport nutrition and exercise metabolism. 2005; 15: 413-24.
6. Alway SE, Pereira SL, Edens NK, et al. beta-Hydroxy-beta-methylbutyrate (HMB) enhances the proliferation of satellite cells in fast muscles of aged rats during recovery from disuse atrophy. Experimental gerontology. 2013; 48: 973-84.
7. Wilkinson DJ, Hossain T, Hill DS, et al. Effects of leucine and its metabolite beta-hydroxy-beta-methylbutyrate on human skeletal muscle protein metabolism. The Journal of physiology. 2013; 591: 2911-23.
8. Giron MD, Vilchez JD, Shreeram S, et al. beta-Hydroxy-beta-methylbutyrate (HMB) normalizes dexamethasone-induced autophagy-lysosomal pathway in skeletal muscle. PloS one. 2015; 10: e0117520.
9. Kimura K, Cheng XW, Inoue A, et al. beta-Hydroxy-beta-methylbutyrate facilitates PI3K/Akt-dependent mammalian target of rapamycin and FoxO1/3a phosphorylations and alleviates tumor necrosis factor alpha/interferon gamma-induced MuRF-1 expression in C2C12 cells. Nutrition research. 2014; 34: 368-74.
10. Eley HL, Russell ST, Tisdale MJ. Attenuation of depression of muscle protein synthesis induced by lipopolysaccharide, tumor necrosis factor, and angiotensin II by beta-hydroxy-beta-methylbutyrate. American journal of physiology Endocrinology and metabolism. 2008; 295: E1409-16.
11. Eley HL, Russell ST, Baxter JH, et al. Signaling pathways initiated by beta-hydroxy-beta-methylbutyrate to attenuate the depression of protein synthesis in skeletal muscle in response to cachectic stimuli. American journal of physiology Endocrinology and metabolism. 2007; 293: E923-31.
12. Eley HL, Russell ST, Tisdale MJ. Mechanism of attenuation of muscle protein degradation induced by tumor necrosis factor-alpha and angiotensin II by beta-hydroxy-beta-methylbutyrate. American journal of physiology Endocrinology and metabolism. 2008; 295: E1417-26.
13. Pimentel GD, Rosa JC, Lira FS, et al. beta-Hydroxy-beta-methylbutyrate (HMbeta) supplementation stimulates skeletal muscle hypertrophy in rats via the mTOR pathway. Nutrition & metabolism. 2011; 8: 11.
14. Lieberman HR, Bathalon GP, Falco CM, et al. Severe decrements in cognition function and mood induced by sleep loss, heat, dehydration, and undernutrition during simulated combat. Biological psychiatry. 2005; 57: 422-9.
15. Nindl BC, Leone CD, Tharion WJ, et al. Physical performance responses during 72 h of military operational stress. Medicine and science in sports and exercise. 2002; 34: 1814-22.
16. McClung JP, Martini S, Murphy NE, et al. Effects of a 7-day military training exercise on inflammatory biomarkers, serum hepcidin, and iron status. Nutrition journal. 2013; 12: 141.
17. Nindl BC, Scofield DE, Strohbach CA, et al. IGF-I, IGFBPs, and inflammatory cytokine responses during gender-integrated Israeli Army basic combat training. Journal of strength and conditioning research / National Strength & Conditioning Association. 2012; 26 Suppl 2: S73-81.
18. Hoffman JR, Gepner Y, Stout JR, et al. beta-Hydroxy-beta-methylbutyrate attenuates cytokine response during sustained military training. Nutrition research. 2016; 36: 553-63.
19. Kraemer WJ, Hatfield DL, Comstock BA, et al. Influence of HMB supplementation and resistance training on cytokine responses to resistance exercise. Journal of the American College of Nutrition. 2014; 33: 247-55.
20. Fuller JC, Sharp RL, Angus HF, et al. Comparison of availability and plasma clearance rates of beta-hydroxy-beta-methylbutyrate delivery in the free acid and calcium salt forms. The British journal of nutrition. 2015; 114: 1403-9.
21. Gareau MG, Sherman PM, Walker WA. Probiotics and the gut microbiota in intestinal health and disease. Nature reviews Gastroenterology & hepatology. 2010; 7: 503-14.
22. Sanders ME. Probiotics: definition, sources, selection, and uses. Clinical infectious diseases : an official publication of the Infectious Diseases Society of America. 2008; 46 Suppl 2: S58-61; discussion S144-51.
23. Wang Y, Gu Q. Effect of probiotic on growth performance and digestive enzyme activity of Arbor Acres broilers. Research in veterinary science. 2010; 89: 163-7.
24. Nissen S, Van Koevering M, Webb D. Analysis of beta-hydroxy-beta-methyl butyrate in plasma by gas chromatography and mass spectrometry. Analytical biochemistry. 1990; 188: 17-9.
25. Froeling M, Oudeman J, Strijkers GJ, et al. Muscle changes detected with diffusion-tensor imaging after long-distance running. Radiology. 2015; 274: 548-62.
26. Deux JF, Malzy P, Paragios N, et al. Assessment of calf muscle contraction by diffusion tensor imaging. European radiology. 2008; 18: 2303-10.
27. Damon BM, Ding Z, Anderson AW, et al. Validation of diffusion tensor MRI-based muscle fiber tracking. Magnetic resonance in medicine. 2002; 48: 97-104.
28. Vickers AJ. The use of percentage change from baseline as an outcome in a controlled trial is statistically inefficient: a simulation study. BMC medical research methodology. 2001; 1: 6.
29. Fuller JC, Jr., Sharp RL, Angus HF, et al. Free acid gel form of beta-hydroxy-beta-methylbutyrate (HMB) improves HMB clearance from plasma in human subjects compared with the calcium HMB salt. The British journal of nutrition. 2011; 105: 367-72.
30. Bouillon R, Van Cromphaut S, Carmeliet G. Intestinal calcium absorption: Molecular vitamin D mediated mechanisms. Journal of cellular biochemistry. 2003; 88: 332-9.
31. Honda H, Gibson GR, Farmer S, et al. Use of a continuous culture fermentation system to investigate the effect of GanedenBC30 (Bacillus coagulans GBI-30, 6086) supplementation on pathogen survival in the human gut microbiota. Anaerobe. 2011; 17: 36-42.
32. Portal S, Zadik Z, Rabinowitz J, et al. The effect of HMB supplementation on body composition, fitness, hormonal and inflammatory mediators in elite adolescent volleyball players: a prospective randomized, double-blind, placebo-controlled study. European journal of applied physiology. 2011; 111: 2261-9.
33. Fischer CP. Interleukin-6 in acute exercise and training: what is the biological relevance? Exercise immunology review. 2006; 12: 6-33.
34. Welc SS, Clanton TL. The regulation of interleukin-6 implicates skeletal muscle as an integrative stress sensor and endocrine organ. Experimental physiology. 2013; 98: 359-71.
35. Febbraio MA, Steensberg A, Keller C, et al. Glucose ingestion attenuates interleukin-6 release from contracting skeletal muscle in humans. The Journal of physiology. 2003; 549: 607-12.
36. Lundeland B, Gundersen Y, Opstad PK, et al. One week of multifactorial high-stress military ranger training affects Gram-negative signalling. Scandinavian journal of clinical and laboratory investigation. 2012; 72: 547-54.
37. Henning PC, Scofield DE, Spiering BA, et al. Recovery of endocrine and inflammatory mediators following an extended energy deficit. The Journal of clinical endocrinology and metabolism. 2014; 99: 956-64.
38. Peterson JM, Feeback KD, Baas JH, et al. Tumor necrosis factor-alpha promotes the accumulation of neutrophils and macrophages in skeletal muscle. Journal of applied physiology. 2006; 101: 1394-9.
39. Wojdasiewicz P, Poniatowski LA, Kotela A, et al. The chemokine CX3CL1 (fractalkine) and its receptor CX3CR1: occurrence and potential role in osteoarthritis. Archivum immunologiae et therapiae experimentalis. 2014; 62: 395-403.
40. van Zuiden M, Kavelaars A, Amarouchi K, et al. IL-1beta reactivity and the development of severe fatigue after military deployment: a longitudinal study. Journal of neuroinflammation. 2012; 9: 205.
41. Suzuki K, Nakaji S, Yamada M, et al. Systemic inflammatory response to exhaustive exercise. Cytokine kinetics. Exercise immunology review. 2002; 8: 6-48.
42. Nyangale EP, Farmer S, Cash HA, et al. Bacillus coagulans GBI-30, 6086 Modulates Faecalibacterium prausnitzii in Older Men and Women. The Journal of nutrition. 2015; 145: 1446-52.
43. Reikeras O. Immune depression in musculoskeletal trauma. Inflammation research : official journal of the European Histamine Research Society [et al]. 2010; 59: 409-14.
44. Lazarus JJ, Kay MA, McCarter AL, et al. Viable Borrelia burgdorferi enhances interleukin-10 production and suppresses activation of murine macrophages. Infection and immunity. 2008; 76: 1153-62.
45. Peake JM, Della Gatta P, Suzuki K, et al. Cytokine expression and secretion by skeletal muscle cells: regulatory mechanisms and exercise effects. Exercise immunology review. 2015; 21: 8-25.
46. Tidball JG, Villalta SA. Regulatory interactions between muscle and the immune system during muscle regeneration. American journal of physiology Regulatory, integrative and comparative physiology. 2010; 298: R1173-87.
47. Paulsen G, Mikkelsen UR, Raastad T, et al. Leucocytes, cytokines and satellite cells: what role do they play in muscle damage and regeneration following eccentric exercise? Exercise immunology review. 2012; 18: 42-97.
48. Kanda K, Sugama K, Hayashida H, et al. Eccentric exercise-induced delayed-onset muscle soreness and changes in markers of muscle damage and inflammation. Exercise immunology review. 2013; 19: 72-85.
49. Cermak NM, Noseworthy MD, Bourgeois JM, et al. Diffusion tensor MRI to assess skeletal muscle disruption following eccentric exercise. Muscle & nerve. 2012; 46: 42-50.

## Claims

1. A non-therapeutic method for maintaining muscle integrity in an animal in need thereof, the method comprising administering a composition comprising from about 0.5 g to about 30 g of β-hydroxy-β-methylbutyric acid (HMB) and at least one probiotic to the animal, wherein the animal is a human or a non-human mammal, wherein the probiotic is *Bacillus coagulans.*

2. The non-therapeutic method of claim 1, wherein said HMB is a calcium salt or wherein HMB is in the free acid form.

3. Non-therapeutic use of a composition for use in maintaining muscle integrity in an animal in need thereof, the composition comprising from about 0.5 g to about 30 g of β-hydroxy-β-methylbutyric acid (HMB) and at least one probiotic, wherein the composition is to be administered to the animal, wherein the animal is a human or a non-human mammal, wherein the probiotic is *Bacillus coagulans.*

4. The non-therapeutic use of claim 3, wherein said HMB is a calcium salt or wherein HMB is in the free acid form.

## Patentansprüche

1. Nicht-therapeutisches Verfahren zum Aufrechterhalten von Muskelintegrität in einem entsprechend bedürftigen Lebewesen, das Verfahren umfasst das Verabreichen einer Zusammensetzung, die von etwa 0,5 g bis etwa 30 g β-Hydroxy-β-methylbuttersäure (HMB) und mindestens ein Probiotikum umfasst, an das Lebewesen, wobei das Lebewesen ein Mensch oder ein nicht-menschliches Säugetier ist, wobei das Probiotikum *Bacillus coagulans* ist.

2. Nicht-therapeutisches Verfahren nach Anspruch 1, wobei das HMB ein Calciumsalz ist oder wobei HMB in Form der freien Säure vorliegt.

3. Nicht-therapeutische Verwendung einer Zusammensetzung zur Verwendung zum Aufrechterhalten von Muskelintegrität in einem entsprechend bedürftigen Lebewesen, die Zusammensetzung umfasst von etwa 0,5 g bis etwa 30 g β-Hydroxy-β-methylbuttersäure (HMB) und mindestens ein Probiotikum, wobei die Zusammensetzung an das Lebewesen verabreicht wird, wobei das Lebewesen ein Mensch oder ein nicht-menschliches Säugetier ist, wobei das Probiotikum *Bacillus coagulans* ist.

4. Nicht-therapeutisches Verwendung nach Anspruch 3, wobei das HMB ein Calciumsalz ist oder wobei HMB in Form der freien Säure vorliegt.

## Revendications

1. Méthode non thérapeutique pour maintenir l'intégrité musculaire chez un animal en ayant besoin, la méthode comprenant l'administration d'une composition comprenant d'environ 0,5 g à environ 30 g d'acide β-hydroxy-β-méthylbutyrique (HMB) et d'au moins un probiotique à l'animal, l'animal étant un mammifère humain ou non humain, le probiotique étant le *Bacillus coagulans.*

2. Méthode non thérapeutique selon la revendication 1, dans laquelle ledit HMB est un sel de calcium ou dans laquelle le HMB est sous forme d'acide libre.

3. Utilisation non thérapeutique d'une composition à utiliser pour maintenir l'intégrité musculaire chez un animal en ayant besoin, la composition comprenant d'environ 0,5 g à environ 30 g d'acide β-hydroxy-β-méthylbutyrique (HMB) et au moins un probiotique, la composition devant être administrée à l'animal, l'animal étant un mammifère humain ou non humain, le probiotique étant le *Bacillus coagulans.*

4. Utilisation non thérapeutique selon la revendication 3, dans laquelle ledit HMB est un sel de calcium ou dans laquelle le HMB est sous forme d'acide libre.
